# EUROPEAN PATENT SPECIFICATION

(11) **EP 0 779 076 B1**
(45) Date of publication and mention of the grant of the patent: **09.03.2005**
(21) Application number: 95924554.9
(22) Date of filing: 27.06.1995
(51) Int. Cl.: C07K 5/10, A61K 38/00, C07K 7/06

(54) **PEPTIDE, A METHOD OF OBTAINING IT AND A PHARMACEUTICAL COMPOUND BASED ON IT**
EIN PEPTID, EIN VERFAHREN ZU SEINER DARSTELLUNG UND EIN AUF IHM BASIERENDE PHARMAZEUTISCHE ZUBEREITUNG
PEPTIDE, SON PROCEDE D'OBTENTION ET COMPOSE PHARMACEUTIQUE BASE SUR LEDIT PEPTIDE

(30) Priority: 29.06.1994 RU 94024278
(43) Date of publication of application: 18.06.1997
(73) Proprietor: Immunotech Developments Inc., Toronto, Ontario M2N 4J2 (CA)
(72) Inventor: DEIGIN, Vladislav Isakovich, Toronto, Ontario M2N 4J2 (CA); YAROVA, Elena Petrovna, Richmond Hill, Ontario L4C 6R8 (CA)
(74) Representative: Benedum, Ulrich Max, Dr.
(86) International application number: PCT/RU1995/000138
(87) International publication number: WO 1996/002267

(56) References cited:
- EP-A- 0 106 404
- EP-A- 0 128 097
- EP-A- 0 134 582
- EP-A- 0 143 850
- WO-A-86/02079
- WO-A-90/06946
- WO-A-90/06947
- WO-A-94/15959
- GB-A- 2 070 618
- CHEMICAL ABSTRACTS, vol. 124, no. 21, 20 May 1996 Columbus, Ohio, US; abstract no. 279588, FLEISHMAN, M. YU. ET AL: "Effect of synthetic analogs of dermorphin on epithelial cell division in white rat cornea and tongue" XP002089772 & BYULL. EKSP. BIOL. MED. ( 1994 ), 118(11), 508-10 CODEN: BEBMAE;ISSN: 0365-9615,1994,
- CHEMICAL ABSTRACTS, vol. 122, no. 7, 13 February 1995 Columbus, Ohio, US; abstract no. 72296, AMBO, AKIHIRO ET AL: "Synthesis and opioid receptor affinity of dermorphin tetrapeptide analogs with acidic amino acid at position 2" XP002089773 & ANNU. REP. TOHOKU COLL. PHARM. ( 1993 ), 40, 169-73 CODEN: TYKNAQ;ISSN: 0495-7342, 1993,
- CHEMICAL ABSTRACTS, vol. 122, no. 7, 13 February 1995 Columbus, Ohio, US; abstract no. 81927, SASAKI, YUSUKE ET AL: "Synthesis and biological properties of quaternized N-methylation analogs of D-Arg-2-dermorphin tetrapeptide" XP002089774 & BIOORG. MED. CHEM. LETT. ( 1994 ), 4(16), 2041-4 CODEN: BMCLE8;ISSN: 0960-894X,1994,
- FLEISHMAN M.Y. ET AL: 'Effect of Synthetic Analogs of Dermorphin on Mitosis in the Corneal and Lingual Epithelium of Albino Rats' BULLETIN OF EXPERIMENTAL BIOLOGY AND MEDICINE vol. 118, no. 11, November 1994, pages 1205 - 1207, XP001098714
- SASAKI Y. ET AL: 'Synthesis and Biological Properties of Quaternized N-Methylation Analogs of D-Arg-2-Dermorphin Tetrapeptide.' BIOORGANIC *A MEDICINAL CHEMISTRY LETTERS vol. 4, no. 16, 25 August 1994, pages 2049 - 2054, XP001007332
- SCHILLER P.W. ET AL: 'DALDA: An extremely mu receptor-selective dermorphin analog capable of inducing peripheral antinociception.' PEPTIDES: CHEMISTRY, STRUCTURE AND BIOLOGY 1990, pages 323 - 325, XP001096293
- AMBO A. ET AL: 'Synthesis and Opioid Receptor Affinity of Dermorphin Tetrapeptide Analogs with Acidic Amino Acid at Position 2' TOHOKU YAKKA DAIGAKU KENKYU NEMPO (ANNU REP TOHOKU COLL PHARM) vol. 40, 1993, pages 169 - 173, XP001007300

## Description

The present invention relates to new peptides and derivatives thereof, a method of obtaining them and to pharmaceutical compositions with those peptides and derivatives.

The present invention concerns the fields of chemistry, medicine, and veterinary medicine, and in particular the field of biologically active peptides. It relates to new pharmaceutical compositions with those peptides and derivatives and to methods of obtaining them. The newly developed compounds can be used for their wide spectrum of biological activities as they affect and stimulate biological processes as the growth of tissue mass, the activity of the epithelium growth zone, hair growth, the healing of wounds and scars, and anabolic processes. Furthermore, the new compounds have an analgesic activity.

It is well known that all metabolic processes in young or mature organisms are governed by hormones and that peptides may have hormonal activity like other regulating hormones which control some functions in living organisms. However, the number of naturally occurring peptides of this type is limited. Many laboratories have therefore developed methods for the synthesis of peptides which may have a higher biological activity than their natural counterparts (EP 0136720, 1984; EP 0137904, 1984). Furthermore, peptides have been synthesised which are not found in living organisms but have unique properties. Among those peptides, there is a group of peptides which can regulate the hormones which are responsible for growth. There is one peptide which increases the level of growth hormone in the blood (WO 89/07111, 1989; WO 91/16923, 1991) and there are others which decrease the level of growth hormone in the blood (FR 2 235 701, 1978; FR 2 532 308, 1982). Recently, a peptide has been synthesised that increases the level of development of the epidermic layer in humans (WO 90/13570, 1990). Another metallopeptide can stimulate hair growth in warm blooded animals (WO 91/07431, 1991) and a synthetic version thereof produces hair loss in animals (FR 2 487 677, 1982). Thus, it is obviously possible to regulate various metabolic processes by various compositions and peptides. However, it is more desirable to find compositions and substances that produce a specific effect.

Currently, there is only a small number of peptides which have multi-functional activities such as the synthetic peptides described in U.S. 4 680 283 (1987) or GB 2 070 618 (1980) which have a wide spectrum of useful activities and proven to be a biostimulator. When administered or applied it has effects on the anlagen similar to morphines.

The synthetic peptides with opium-like effects have been heavily examined and studied. Unfortunately, the morphine-effect producing combinations are not common in nature and it is very difficult to reproduce them or to find and synthesize those peptides (U.S. 4 042 682, 1977). This situation has been stimulant for the development of a large arsenal of synthetic peptide structures (U.S. 4 681 871, 1987; EP 0 112 036, 1983; EP 0 221 019, 1986).

. A number of opium-like peptides have been widely used in clinical treatments of ischemia of the brain (DE 34 47 720, 1985; U.S. 4 684 624, 1987), as a pain relief for pregnant women (EP 0 099 173, 1984) and as analgesic compounds which are especially effective when used in conjunction with a traditional therapy (EP 0 096 592, 1983; EP 0 099 286, 1984; U.S. 4 123 523, 1978; Annu. Rep. Tohoku Coll. Pharm. (1993), 40, 169-73).

WO 86/02079) discloses other peptides of the formula H-Tyr-A-Phe-Val-T, wherein A is any L-proline or D-amino acid and T is OH, NH₂ or NHNHR and which are said to be useful in treating diseases.

Moreover, Schiller P.W. et al. describe in Peptides: Chemistry, Structur and Biology (1990), 323-325 a peptide of the formula H-Tyr-D-Arg-Phe-D-Lys-NH₂ which has µ receptor affinity.

Biologically active tetra and penta peptides have similarily been described in NZ 194,961 (GB 2 070 618) which peptides contain in the core structure neutral L-amino acid residues.

Notwithstanding, the known synthetic peptides and compositions can not fully satisfy the demands of modern medicine as they either have not the necessary properties or are not effective enough.

Methods on basis of those peptides require large dosages to obtain the desired effect, at the same time raising the danger of unwished side effects.

The discovered peptides are provided for experimental and medical purposes. The present peptides and compositions can be used as opium-like substances and for similar purposes. The present peptides can be used as a substance with anabolic properties, for increasing the mass of living tissue, for effecting growth, in particular the growth of skin and hair. The biostimulating properties of the present peptides are well suited to stimulate for repair processes and wound healing.

To obtain the peptides in question, modern effective peptide synthesis methods are available which allow the use of minimal amounts of material and only require a limited number of steps to obtain large amounts of product.

The peptides of the present invention can be described by the following formula I: X-Tyr-Y-Phe-Z-A wherein
- X: is Arg, D-Arg, Orn, D-Orn, Lys, D-Lys, Har, D-Har, Cyt, D-Cyt;
- Y: is D-Ala, D-Val, D-Leu, D-Ile, D-Phe, D-Asn, D-Trp, D-Pro, D-Ser, D-Thr, D-Tyr, D-Hyp, D-Cys, D-Cys-Cys, D-Met, D-Lys, D-Har, D-Arg, D-His, D-Asp, D-Glu, D-β-Ala, D-Orn;
- Z: is D-Ala, D-Val, D-Leu, D-Ile, D-Phe, Asn, D-Asn, Gly, Gln, D-Gln, D-Trp, D-Pro, D-Ser, D-Thr, D-Tyr, Hyp, D-Hyp, Cys, D-Cys, Cys-Cys, Cys-D-Cys, D-Cys-Cys, D-Cys-D-Cys, D-Met, Lys, D-Lys, Arg, D-Arg, His, D-His, Asp, D-Asp, Glu, D-Glu, β-Ala, D-β-Ala, Orn, D-Orn;
- A: is OH or a substituted amide (C₁ - C₃);
and wherein Arg is arginine, Orn is ornithine, Lys is lysine, Har is homoarginine, Cyt is citrulline, Ala is alanine, Val is valine, Leu is leucine, Ile is isoleucine, Phe is phenylalanine, Asn is asparagine, Trp is tryptophan, Pro is proline, Ser is serine, Thr is threonine, Tyr is tyrosine, Hyp is hydroxyproline, Cys is cysteine, Cys-Cys is cystine, Met is methionine, His is histidine, Asp is aspartic acid, Glu is glutamic acid, Gly is glycine, and Gln is glutamine. The peptides of formula I can be derived by a stepwise construction of the peptide chain beginning at the C-end section of the amino acid Z, which is a substituted amide or protected carboxylic group, followed by the binding of an N-protected phenylalanine with the method of mixed anhydrides or activated ether. The next steps are the removal of the N-protected group, the addition of activated esters or mixed anhydrides, followed by the N-protected amino acid, the removal of the N-protected group and the addition of the N-protected amino acid. When the chain has been completed, a tetra or pentapeptide is released, the peptide product being obtained by a hydrogenation step in the presence of a palladium catalyst.

The above-mentioned peptide product is obtained as a white powder which may have a shade of yellow or grey and which is soluble in water. It does dissolve well in alcohol and is insoluble in chloroform.

Table 1 shows the physical-chemical properties of peptides of formula I. "R_{fB}" was measured in a solution of butanol, acetic acid, water in a ratio of 3:1:1. "R_{fA}" was determined in a solution of chloroform, methanol, and 32% acetic acid in a ratio of 60:45:20.

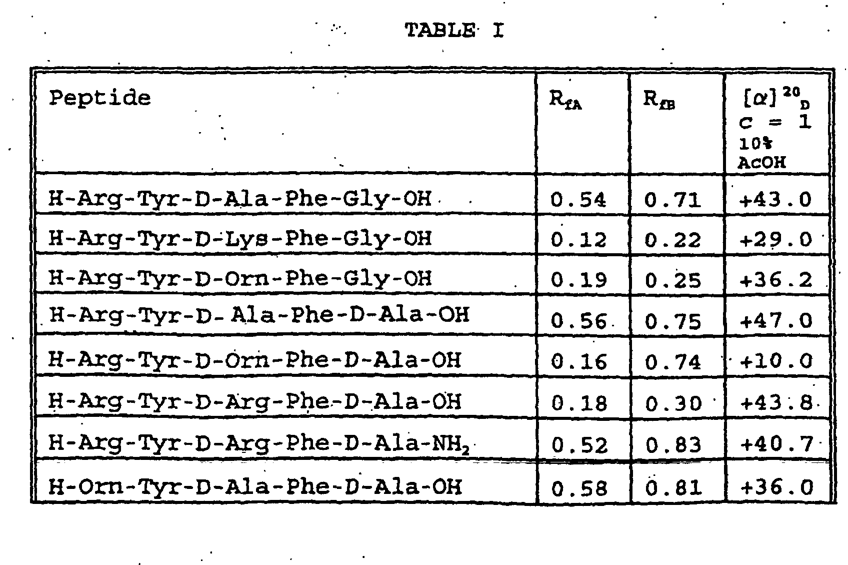

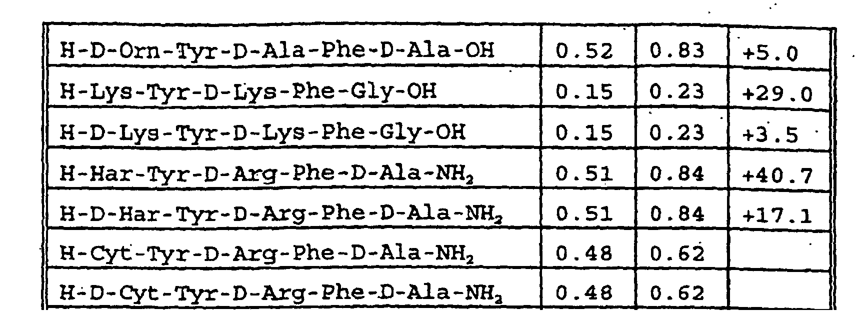

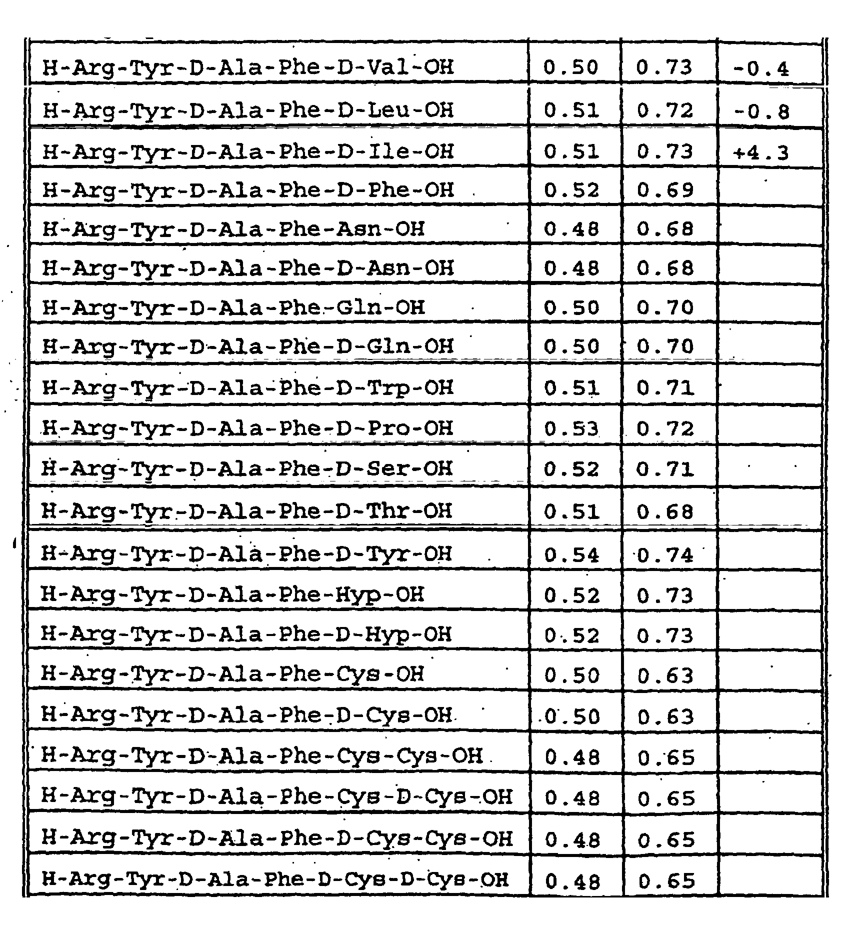

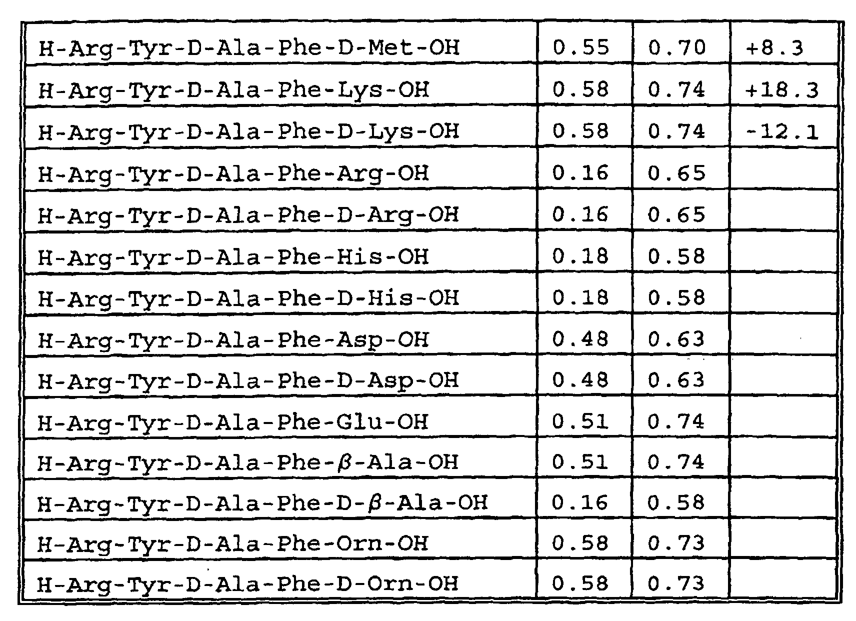

The peptides of the present invention can be used as a biologically active ingredient for the preparation of pharmaceutical compositions or medicaments. The pharmaceutical compositions may include an effective quantity of the peptide as well as a suitable carrier or diluent. The composition can be used as a medicament or food supplement.

The composition can be formulated for oral administration or for external or topical use as well as for intraperitoneal administration as an injection.

The composition has usually a peptide content from 0.001 to 0.1% (preferable 0.001 to 0.01%). The amount of peptide also depends on how much water is in the liquid or solid substance. In order to obtain a pharmaceutical composition it is necessary to mix the peptide with the carrier at a temperature in range between 40 and 70 °C. The mixture is also stable in solution over 24 hrs (3 years) at a temperature of 70°C (20°C).

For injection purposes, any pharmaceutical solvent can be used including distilled water, a physiological solution and a buffer solution. The peptide can be present in the solvent from 0.001% to 0.01% by weight.

The injection solution can be prepared by a traditional weight volume method. Depending on the amount of the peptide powder by weight, a proper amount of solvent is added to obtain the required mixture for injection purposes. The mixture is then filtered through a sterilizing filter system and split into test tubes or ampules. The resulting mixture is a stable clear liquid without any chemical or other additives.

The solid form of the peptide for oral intake can be that of tablets, powder or capsules which can also be used as a base for other additives such as colouring and flavouring additives. The ratio between the peptide and the additive can be from 0.001% to 0.01% according to the purpose of the mixture.

When investigating the biological functions of the new peptides, it was discovered that the new peptide can be used for their wide variety of activities.

It was found that the new peptides are not toxic. To determine the level of LD₅₀ toxicity of the peptide, 24 white mice weighing 18 g each were tested with identical living and feeding conditions for the duration of experiment. In all test groups the mice comprised 50% males and 50% females. The mice were kept in a vivarium quarters with constant temperature and ventilation 24 hours prior and during the entire experiment. The feeding and watering of the mice was stopped 2 hours prior to the beginning of the experiment. The mice were divided into 4 groups of 6. Three groups of mice received intraperitoneal injections of 0.2 ml of a mixture of peptide and water at a dosage of 800, 1100, 1400 µg/kg. The fourth control group received injections of physiological solution in the same quantity. The mice were then monitored for 7 days and nights during which time the LD₅₀ values were obtained. During the experiment no changes in behaviour and condition of the test animals were detected and the injection of the peptide was not followed by any syndromes of illness. No deaths occurred during the duration of the experiment and all animals gained weight normally. The weights of the organs were within normal limits. The results show that injections with the above indicated doses of peptides did not harm the animals and had no toxicity.

The following examples describe preferred methods of deriving peptides of formula I

### Example 1 - Synthesis of H-Arg-Tyr-D-Ala-Phe-Gly-OH.

### A) Preparation of BOC-Phe-Gly-oBzl

5.6 g (40.0 mmol) Isobutylchloroformate was added to a stirred solution of 10.7 g (40 mmol) Boc-Phe-OH and 4.8 ml (40.1 mmol) N-methylmorpholine in 50 ml dimethylformamide which was cooled to -15° C. To this cooled mixture a chilled solution was added after 2 minutes which contained p-toluenesulfonate benzylesters of glycine in 50 ml dimethylformamide. The reaction mixture was stirred for 30 min at - 15 C and then for 2 hours at room temperature. The dimethylformamide was distilled under vacuum and to the residue 100 ml ethylacetate added. The mixture was washed twice with 50 ml of 2% solution of sulfuric acid which was rinsed twice with 80 ml of saturated solution of sodium bicarbonate, washed with water to neutral reaction and then dried over anhydrous sodium sulfate.

The ethylacetate layer was distilled under vacuum. The residue was recrystallized from ethylacetate by adding ether and hexane. The yield was 16.4 g (98.2%). Tₘₚ: 135.2°C; R_{fA}: 0.71 in a mixture of chloroform/ethylacetate/methanol (3:6:1). R_{fB}: 0.62 in a mixture of ethylacetate/hexane (1:1).

### B) Preparation of BOC-D-Ala-Phe-Gly-Obzl

16.4 g (39.8 mmol) of BOC-Phe-Gly-oBzl was dissolved in 80 ml of 50% trifluoroacetic acid in chloroform. After one hour, the solvents were evaporated under vacuum until it a solid oil was formed. 150 ml of diethyl ether was added to the residue and the residue recrystallized. The residue was filtered, washed with ether and dried. The yield was 16.2 g (99.8%). Tₘₚ: (TFA·H-Phe-Gly-oBzl) 135°C; R_{f}: 0.34 in a mixture of chloroform/methanol = 9:1.

The mixture of 7.5 g (39.7 mmol) BOC-D-Ala-OH and 4.6 mg (40.0 mmol) N-methylmorpholine in 50 ml dimethylformamide was then cooled to -15 °C and while mixing the solution 5.6 g (40.3 mmol) isobutylchloroformate was added. After 2 minutes, a cooled mixture of 16.2 g (40.0 mmol) TFA·H-Phe-Gly-oBzl and 4.6 ml (40.0 mmol) N-methylmorpholine in 50 ml of dimethylformamide was added and the reaction mixture was stirred for 30 min at -15°C and 2 hours at room temperature. Dimethylformamide was removed under vacuum and to the residue 100 ml of ethylacetate was added and twice washed with a 2% solution of sulfuric acid (50 ml each time), twice washed with 80 ml sodium bicarbonate solution each time, then washed with water until a neutral reaction, and finally dried over anhydrous sodium sulfate. The ethylacetate layer was evaporated under vacuum and the residue recrystallized from ether. Yield: 14.5 g (75.9%); Tₘₚ: 140°C; R_{f}: 0.79 in a mixture of chloroform/ethylacetate/methanol (6:3:1); R_{f}: 0.55 in a mixture of chloroform/methanol (9:1).

### C) Preparation of BOC-Tyr-(BOC)-D-Ala-Phe-Gly-oBzl

14.4 g (29.8 mmol) BOC-D-Ala-Phe-Gly-oBzl was dissolved in 80 ml of 50% trifluoroacetic acid in chloroform.

After one hour the solvents were evaporated until they took the shape of an oily substance. The residue was dissolved in 50 ml of dimethylformamide and 3.4 mg (29.8 mmol) of N-methylmorpholine added. The mixture of 11.4 g (30.0 mmol) BOC-Tyr-(BOC)-OH- and 3.45 ml (30.0 mmol) N-methylmorpholine in 50 ml of dimethylformamide, was cooled to -15°C and while mixing 4.27 g of (30.0 mmol) isobutylchloroformate added. After 2 minutes, to this mixture was added 14.3 g (mmol) benzylether tripeptide: H-D-Ala-Phe-Gly-oBzl. This reaction mixture was mixed over 30 minutes at a temperature of -15°C and for 2 hours at room temperature. Dimethylformamide was removed under vacuum and to the residues 100 ml of ethylacetate added, which was then twice washed with a 2% solution of 50 ml sulphuric acid, thereafter twice washed with an enriched mixture of 80 ml of bicarbonate sodium. The remains were then washed in water to achieve a neutral reaction and dried over sulfate sodium. The layer of ethylacetate was then evaporated under vacuum and the remains recrystallized. Yield: 18.5 g (83.4%): Tₘₚ: 133.5°C; R_{f}: 0.62 in a mixture of chloroform/ethylacetate/methanol (6:3:1); R_{f}: 0.57 in a mixture of chloroform/methanol (9:1).

### D) Preparation of BOC-Arg(NO₂)-Tyr-D-Ala-Phe-Gly-oBzl

9.8 g (13.1 mmol) BOC-Tyr-(BOC)-D-Ala-Phe-Gly-oBzl was dissolved in 80 ml of a 50% solution of trifluoroacetic acid in chloroform. After one hour the solvents were evaporated until an oily substance was obtained. The residue was recrystallized in ether. Yield: 9.68 g (100%).

9.6 g (13.0 mmol) TFA·Tyr-D-Ala-Phe-Gly-oBzl was dissolved in 150 ml dimethylformamide and 1.5 ml (13.0 mmol) N-methylmorpholine added. 5.2 g (14.6 mmol) BOC-Arg(NO₂) OH·1/2THF, 1.97 g (14.6 mmol) oxybenzotriazol. The reaction mixture was cooled while mixing to -10 °C and 3.0 g (14.0 mmol) dicyclohexylcarbodiimide added. The mixture was stirred for 72 hours at room temperature. The dicyclohexylurea was filtered off, the solvent then evaporated under vacuum and to the residue 100 ml of ethylacetate was added, which was washed twice with 50 ml of a 2% solution of sulfuric acid. The mixture was then washed twice with mixture of 80 ml of bicarbonate sodium, then rinsed with water until neutral, and dried with anhydrous sulfate sodium.

The layer of ethylacetate was evaporated under vacuum, and the residue recrystallized. Yield: 9.6 g (87.2%); Tₘₚ: 176.7°C; R_{f}: 0.70 in a mixture of chloroform/ethyl-acetate/methanol/acetic acid (6:3:1:0.5); R_{f}: 0.23 in a mixture of chloroform/methanol (9:1).

### E) Preparation of H-Arg-Tyr-D-Ala-Phe-Gly-OH

9.6 g (13.6 mmol) BOC-Arg(NO₂)-Tyr-D-Ala-Phe-Gly-oBzl was dissolved in 60 ml formic acid and 1.0 g of palladium catalyst added. Hydrogen was added for 6 hours. The catalyst was filtered off and the formic acid evaporated under vacuum with repeated addition of 100 ml of water. The residue was added to diethyl ether. The residue was filtered off, rinsed with ether, and dried. Yield: 6.9 g (100%); R_{f}: 0.44 in a mixture of butanol/acetic acid/water (3:1:1).

Further purification of the peptide was performed by ion exchange chromatography on a column packed with sorbent sephadex™ G-25 and the gradient was 0.1 M to 1.0 M pyridin-acetate buffer mixture.

According to physical-chemical analysis, the peptide H-Arg-Tyr-D-Ala-Phe-Gly-OH has a molecular mass of 612.6, linear structure. It is a white powder with yellow shade which is dissolvable in water, not well dissolvable in alcohol and nonsoluble in chloroform.

The UV spectrum in the range of 250 - 300 nm has a peak at 275 ± 2 nm, a shoulder at 282 ±2 mm; 0.1% water mixture had a pH of 5.0 - 7.0.

### Example 2

This example outlines the method of preparation for the peptide H-Arg-Tyr-D-Orn-Phe-D-Ala-OH.

### A) Preparation of Z-Phe-D-Ala-OH

9.68 g (20.0 mmol) Z-Phe-OPfp was dissolved in 20 ml of tetrahydrofurane and 2.3 g (25.6 mmol) H-D-Ala-OH, dissolved in 5 ml of water pH=8.5, added. The reaction mixture was stirred during 24 hours at room temperature. The solvents were evaporated under vacuum, adding 70 ml ethylacetate and 70 ml of 2% sulphuric acid until a pH of 2 to 3 was reached. The peptide was extracted twice with 70 ml of ethylacetate, washed with saturated sodium chloride until neutral, then dried with anhydrous sodium carbonate. The solvents were removed and evaporated under vacuum until an oily product was obtained.

The product was recrystallized from ether and hexane. Yield: 7.6 g (97.7%); R_{f}: 0.40 in chloroform/ethylacetate/methanol/acetic acid (6:3:1:0.2); R_{f:} 0.53 in acetic acid/chloroform/methanol (0.5:16:1); R_{f}: 0.58 in acetic acid/chloroform/methanol (0.5:9:1); Tₘₚ: 153-154°C; [α]²⁰_{D}=+3.2 (C=1 MeOH).

### B) Preparation BOC-D-Orn(Z)-Phe-D-Ala-OH.

6.5 g (18.0 mmol) Z-Phe-D-Ala-OH was dissolved in 15 ml of methanol and 2 ml of formic acid and 0.2 g of palladium-carbon added. Hydrogen was passed through for 3 hours. The catalyst was filtered off and the solvents evaporated under vacuum. The remains were dosed with ether. The residue was filtered, washed with ether and dried. Yield (H-Phe-D-Ala-OH): 4.3 g (98.1%); R_{f}: 0.01 in chloroform/ethylacetate/methanol/acetic acid (6:3:1:0.1); R_{f}: 0.45 in acetic acid/chloroform/methanol (1:3:2).

8.0 g (15.0 mmol) pentafluorophenyl ester Boc-D-Orn(Z)-OPfp was dissolved in 15 ml of dioxane and while mixing 4.3 g (17.7 mmol) H-Phe-D-Ala-OH added, which was dissolved in water of pH 8.5. The reaction mixture was stirred at room temperature for 24 hours. The solvents were evaporated under vacuum and processed with same method as in paragraph (a). The resulting mixture was recrystallized from ethylacetate/ether/hexane. Yield: 6.92:g (74.4%); R_{f}: 0.44 in acetic acid/chloroform/methanol (0.5:16:1); R_{f}: 0.32 in chloroform/ethylacetate/methanol (6:3:1); Tₘₚ: 145 °C.

### C) Preparation of BOC-Tyr-(Bzl)-D-Orn(Z)-Phe-D-Ala-OH

3.3 g (5.6 mmol) BOC-D-Orn(Z)-Phe-D-Ala-OH was dissolved in 10 ml of 50% trifluoroacetic acid in chloroform. After one hour the solvents were evaporated under vacuum and to the residue ether added. The product was filtered, washed with ether and dried. Yield: 3.0 g (100%).

3.0 g (5.4 mmol) BOC-Tyr(Bzl)-OPfp was dissolved in 10 ml dimethylformamide and 3.0 g (5.8 mmol) TFA·H-D-Orn(Z) -Phe-D-Ala-OH and 0.25 ml (5.7 mmol) diethylisopropylamine added.

The reaction mixture was stirred at room temperature for 24 hours. After 24 hours the solvents were removed by evaporation under vacuum and 70 ml of ethylacetate and 70 ml of 2% of sulfuric acid of pH=2-3 added. The peptide was aciolified with 70 ml of ethylacetate, washed with saturated sodium chloride until a neutral and dried with anhydrous sulfate sodium. Then the solvents were removed under vacuum until an oily material was formed. The residue was then recrystallized from ethylacetate and ether. Yield: 4.1 g (82.0%); R_{f}: 0.47 in acetic acid/chloroform/methanol (0.5:16:1); R_{f}: 0.35 in acetic acid/chloroform/methanol (0.5:16:1).

### D) Preparation Z3-Arg-Tyr(Bzl)-D-Orn(Z)-Phe-D-Ala-OH

0.9 g (1.1 mmol) BOC-Tyr (Bzl) -D-Orn (Z) -Phe-D-Ala-OH was dissolved in 5 ml of 50% trifluoroacetic acid in chloroform. After one hour the solvents were removed by evaporation under vacuum and ether added. The residue was filtered, washed with ether and dried. Yield: 0.8 g (100%).

0.65 g (1.1 mmol) Z3-Arg-OPfp was dissolved in 5 ml of dioxane and 1.5 ml dimethylformamide, adding 0.8 g (1.0 mmol) TFA·H-Tyr(Bzl)-D-Orn(Z)-Phe-D-Ala-OH and 0.12 ml (1.0 mmol) diethylisopropylamine. The reaction mixture was stirred at room temperature for 24 hours. The solvents were then removed under vacuum. Ethylacetate was added and the remaining residue filtered. The remains was recrystallized with ethylacetate. Yield: 1.88 g (92.0%); R_{f}: 0.51 in acetic acid/chloroform/methanol (0.5:16:1); R_{f}: 0.34 in chloroform/ethylacetate/methanol/acetic acid (65:3:1:0.1); R_{f}: 0.53 in acetic acid/chloroform/methanol (0.5:9:1); Tₘₚ 138-143 °C.

### E) Preparation of H-Arg-Tyr-D-Orn-Phe-D-Ala-OH.

0.5g (0.39mmol) Z3-Arg-Tyr(Bzl)-D-Orn(Z)-Phe-D-Ala-OH was dissolved in 3 ml of acetic acid and 2 ml of formic acid. 0.62 g of palladium carbon was added and hydrogen passed through for 3 hours. The catalyst was then filtered off and the solvents evaporated under vacuum.

The resulting compound was dissolved in water and purified with reverse phase liquid chromatography on a 1.6 x 25 cm column packed with sorbent "Silasorb™ C-18" (10 mkm) in acetonitrile - 0.01 M triethylammoniumacetate (pH=6.0). Yield: 0.20 g (80.8%).

### Example 3

### Outline of the preparation of a peptide of the following formula: H-Arg-Tyr-D-Arg-Phe-D-Ala-OH.

0.9 g (0.14 mmol) H-Arg-Tyr-D-Orn-Phe-D-Ala-OH guanidile was dissolved in 5 ml of 1N NaOH. The reaction mixture was stirred at room temperature for 7 nights and days and purified by reverse phase liquid chromatography on a 1.6 x 25 cm column packed with sorbent "silasorb C-8"™ (10 mkm) using as gradient acetonitrile to 0.05% trifluoroacetic acid. Yield: 0.087 g (76.9%).

### Example 4 (deleted)

### Example 5

This example describes the various tests for the evaluation of the opium-like activity of those peptide.

Opium-like activity was studied in a classical way by in-vitro tests on isolated organs: - Test GPI (Guang T.A, Kosterlitz H.W, *Agonist and antagonist action of morphine like drugs on the guinea pigs isolated ileum*, Br.J. Pharmacol. 1966, V.27N3.P.514-527 - Test MVD; Hughs J., Kosterlitz H.W., Leslie F.M, *Effect of morphine on adrenergic transmission in mouse is different. Assessment of agonist and antagonist potencies of narcotics*, Br.J. Pharmacol., 1975, V. 53, N3-P. 371-381) As well as tests to determine opium-like activities by the *in-vitro* hot plate test (Aukier S.I., *New hot plate tests to qualify analgesic and narcotic antagonist activities*, Eur.J. Pharmacol., 174 - V.27 Ni p.1-4) and "Tail flick" on rats (D'Amour F.E., Smith D.L, *A method for determining loss*, Exp.Ther.,1974, V.72 Ni-p.74-79).

. The analgesic activities of those peptides were tested by the "tail flick" method on rats which received intranasally a dose of 0.005 mg/kg. A real analgesic effect of 242 units was characteristic to the peptide H-Arg-Tyr-D-Ala-Phe-Gly-OH. Table 2 outlines the findings with regard to the opium-like activity for this peptide group.

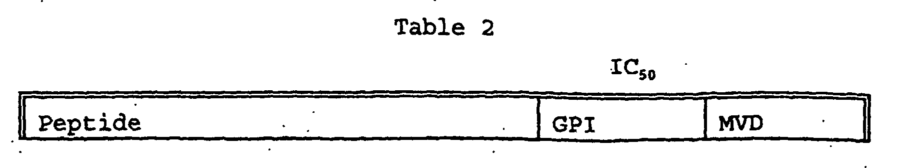

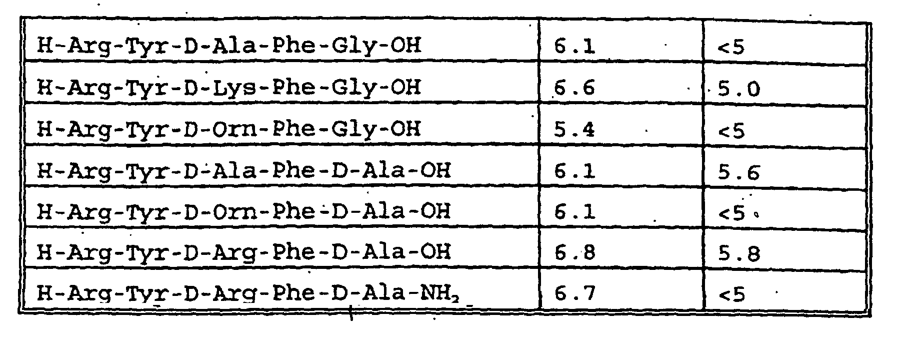

It should be noted that minimal dosage of peptide that causes any kind of biological effect is 1-10 µg/kg.

The biological activities of those peptides and derivatives were tested on mice, rats, mink, birds, fish, young bulls and pigs.

### Example 6

This example describes the effects of the present peptides on the weight gain of young rats and mice.
a) The experiments were conducted on 4 groups of rats of 1 month age. The control group 1 received 0.1 ml injections of physiological solution. The control groups 2, 3, and 4 received the peptide intravenously at a dosage of 0.1, 0.5. 1.0 µg/kg. The rats in all 4 groups were weighed every 5 days while monitoring their food uptake. The experiment lasted for 35 days. The results had an accuracy of <0.05 and are described in table 3.

**Table 3**

| # of test group | dosage of peptide µg/kg | weight gain in % | net weight gain % |
|---|---|---|---|
| 1 | - | 260 | 0 |
| 2 | 0.1 | 270 | 3.8 |
| 3 | 0.5 | 275 | 5.7 |
| 4 | 1.0 | 310 | 19.0 |

The largest weight gain was observed in group 4 which received dosages of 1.0 µg/kg is of peptide.
The following table 4 outlines the total food intake of the rats.

**Table 4**

| # of test group | peptide dosage µg/kg | amount of food g/day/animal |
|---|---|---|
| 1 | - | 14.5 |
| 2 | 0.1 | 15.5 |
| 3 | 0.5 | 14.5 |
| 4 | 1.0 | 16.0 |

The investigation of the food consumption of the various groups shows no differences which indicates that the peptide has anabolic activity.
b) This experiment was performed on 7 groups of one month old mice of NMRI strain. The mice had unrestricted access to food and water. The first group received physiological injections of 0.1 ml, the 2nd, 3rd and 4th groups received injections of peptide at a dosage of 1.0 µg/kg for the 2nd group, 10.0 µg/kg for the third group and 100.0 µg/kg for the fourth group. The 5th, 6th and 7th group received daily during 30 days of experiment dosages of water solution of peptide intravenously at a dosage of 0.1, 0.5 and 1.0 µg/kg. The mice where weighed twice a week during the 30 days experiment while monitoring the daily intake of food.

The results of the weight gains are outlined in the following table 5.

**Table 5**

| # of test group | dose of peptide µg/kg | increase in weight % | net weight gained % |
|---|---|---|---|
| 1 | - | 68 | 0 |
| 2 | 1.0* | 142 | 108 |
| 3 | 10.0* | 105 | 54 |
| 4 | 100.0* | 90 | 32 |
| 5 | 0.1** | 80 | 18 |
| 6 | 0.5** | 70 | 3 |
| 7 | 1.0** | 65 | -0.4 |

| | | | |
|---|---|---|---|
| * - Oral administration | | | |
| ** - Intravenous administration | | | |

The most effective method of administering the peptide is orally by a water solution containing a dosage of 1 µg/kg. The following are average quantity of food consumed for each group as measured every 24 hours.

**Table 6**

| # of test group | dosage of peptide µg/kg | amount of food g/day/animal |
|---|---|---|
| 1 | - | 5.6 |
| 2 | 1.0* | 7.0 |
| 3 | 10.0* | 5.0 |
| 4 | 100.0* | 5.5 |
| 5 | 0.1** | 5.3 |
| 6 | 0.5** | 5.2 |
| 7 | 1.0** | 5.8 |

The findings indicate that the highest weight gain occurred in a group of animals which received the greatest amount of food along with a dosage of peptide of 1.0 µg/kg in a water solution which was administered orally.

It should be noted that the intake of the peptide is followed in the test animals by a gain of weight and an increased food uptake. The observed weight gain from the uptake of 1 gram of food is an indicator for the commercial application of this peptide. The results are shown in table 7.

**Table 7**

| # of test group | dose of peptide µg/kg | net weight gain in % |
|---|---|---|
| 1 | - | 100 |
| 2 | 1.0* | 128.1 |
| 3 | 10.0* | 136.5 |
| 4 | 100.0* | 127 |
| 5 | 0.1** | 110.4 |
| 6 | 0.5** | 115.3 |
| 7 | 1.0** | 97 |

The highest weight gain resulting from the uptake of one gram of food occurred when administering the peptide in a water solution at a dose of 10.0 µg/kg. The net weight gained by the test groups was by 36.5% higher than the weight gained by the control group.

### Example 7

The example describes the influence of the peptides according to the invention on the morphology of the skin and on the growth of hair.

The experiment was conducted on 4 groups of mice of NMRI strain. The first group was the passive control group, the other groups received the peptide at following dosages: 2nd group 1.0 µg/kg, 3rd group 10.0 µg/kg, and 4th group 100.0 µg/kg.

Skin samples of 5 x 5 mm were taken from the crest area of the animals through a biopsy and tested according to "lili" method over 48 hours. The skins were treated in alcohol and immersed in pigment for 24 hours. The next step was a treatment in cellodin. The preparation of histological samples was done with scalpels manufactured by the firm "Rehard". The samples were arranged in an axial order, and the incision edges were coloured using the classic eosin method.

In the histological study, the epidermic and dermic qualities of the 10 µm thick samples were investigated. The number of hair follicles was counted per millimetre according to the "Aftondilov" method in the test and the control groups of the animals.

It has been found that mice receiving 100.0, 10.0, 1.0 µg/kg of peptide had an increase of the hair follicles by 14%, 19% and 22% respectively, in comparison with the control group.

Skin samples of the animal groups revealed that the 4th test group of animals which received 100.0 µg/kg have experienced growth of epidermic layer of hyperkeratosis zones. The third group of animals with a dose of 10.0 µg/kg have experienced changes similar to group # 4. A thickening of the epidermis was also observed. The second test group of animals experienced activity in the growth zone of the epithelial and a better condition of underskin fatty tissue was also observed.

The indicated test groups have experienced the growth of hair on the crest area. Ten hairs from every animal were measured with the accuracy of 1 micron and calculations where made for averages for each test group. The results are shown in table 8.

**Table 8**

| # of test group | doses of peptide µg/kg | increase in length mm | net growth % |
|---|---|---|---|
| 1 | - | 4.2 | 0 |
| 2 | 1 | 4.4 | 4.5 |
| 3 | 10 | 4.9 | 16.7 |
| 4 | 100 | 4.5 | 17.9 |

It should be noted that a peptide administered in doses 10 and 100 µg/kg has a stimulating effect on the growth of hair in mice.

The peptide in these experiments did not show any sign to toxicity. The peptide is also proven to stimulate the growth activity of epithelials, the development of the epidermic skin layer, the growth of density and amount of hair follicles as well as an increasing of the volume of living tissue.

### Example 8

The example outlines the influence of the peptide on the regeneration of the tail fins of fish.

The experiments were conducted on young carp and trout fish. The fish where immersed during 2 months in a peptide solution with a concentration of 1.0 mg/l.

For the duration of the experiment, the regeneration of the tail fins was observed (test and control group were marked by trimming the tail fins for their identification). Upon measurement of the tail fins in the control group it was noted that the length of the regenerated portion was 183+/-23 µm, and the test group showed a measurement of 268+/-26 µm, which is at least 1.5 times greater then the measurement of the control group. The speed of the regeneration process in the test group was also greater by 1.5 times than in the control group of the carp fish.

It is evident that the peptide has growth stimulating properties and can be used for healing purposes.

Upon the treatment of the titer agglutinin erythrocyte of a rabbit in a blood and peptide serum of the carp fish for one week, it was noted that the titer size has increased by 1.95 times.

The conclusion according to the test result is that the fish under the influence of the peptide are more resilient of protozoan and bacterial infection and are better able to cope with external elements.

### Example 9

The example outlines the influence of the peptide on the weight gain in mink and polar fox.

For the purpose of this experiment, young polar fox and mink were used which where 2 months old. There were three test groups and one control group for each type of animal.

The first test group received peptide in a dose of 10 µg/kg of living mass in a form of food supplement.

The second test group received peptide for 10 days in a dose of 10 µg/kg of living mass followed by a 10 day interruption. This continued until the animals where put down.

The third test group received the peptide the same way as the first test group but in dosages of 20 µg/kg of living mass.

After one month from the beginning of the experiment, the animals were weighed. The results showed a difference between males and females of the test groups in comparison to the control group. The results of the experiment, reported as an average, are outlined in table 9.

**Table 9**

| # of test group and type of animal | dosages of peptide as µg/kg | weight increase kg. | | net weight gain % | |
|---|---|---|---|---|---|
| | | female | male | female | male |
| mink | | | | | |
| 1 | - | 0.419 | 0.611 | 100 | 100 |
| 2 | 10 | 0.375 | 0.728 | 90 | 119 |
| 3 | 10 | 0.381 | 0.726 | 91 | 119 |
| 4 | 20 | 0.443 | 0.771 | 106 | 126 |

| polar fox | | | | | |
|---|---|---|---|---|---|
| 1 | - | 0 700 | 0.580 | 100 | 100 |
| 2 | 10 | 1.780 | 0.830 | 105 | 116 |
| 3 | 10 | 1.740 | 1.890 | 102 | 120 |
| 4 | 20 | 1.810 | 1.920 | 106 | 121 |

Results show that the females of both species of animals of the third test group had experienced 6% weight gain when receiving the peptide in a dose of 20 µg/kg of living mass. The mink males had experienced weight gain of 26% and the fox males had experienced 21% weight gain.

### Example 10

The example outlines the influence of the peptide on the improvement of the quality of the furs in mink and polar fox.

The conditions and procedures of this experiment are the same as for experiment 9.

After the animals were put down, the area of the furs was measured and the quality of the fur graded. The increase in area of the furs was noted in all test groups in comparison to the control group while the general quality was maintained at a high level. The increase of the indicators in test group of the mink showed an average of 105% for group # 2, 108% for group # 3, 108% for group # 4. The results for the polar fox where for group # 2 112%, for group # 3 107%, and # 4 106%.

### Example 11

The example outlines the influence of the peptide on weight gain in piglets.

For this experiment, 7 groups of piglets which were weaned from their mother were used. The piglets were from 4 to 5 months old. One group of the piglets served as a control group and the other six where the test groups. The first three test groups received peptide in a form of food supplement at a rate of 1.0 µg/kg for the second test group, 5.0 µg/kg for the third, and 10.0 mkg/kg for the fourth test group in relation to the living mass of the animals. The dosages where administered for 20 days of each month with 10 days each month of interruption. The other three test groups received the peptide in an injection form at a dosage of 0.01% of water solution during 5 days each month with a 25 day interruption. Group # 5 received 0.1 µg/kg, group # 6 received 0.5 µg, and group # 7 received 1.0 µg/kg of living mass of the animal. The experiment lasted for 100 days. The results of the experiment are outlined in table 10.

**Table 10**

| # of test group | dose of peptide µg/kg | weight gain kg | net weight gain % |
|---|---|---|---|
| 1 | - | 52+-3.9 | 100 |
| 2 | 1.0* | 60+-5.1 | 115 |
| 3 | 5.0* | 56+-4.8 | 108 |
| 4 | 10.0* | 68+-4.9 | 130 |
| 5 | 0.1** | 57+-3.7 | 109 |
| 6 | 0.5** | 59+-4.1 | 114 |
| 7 | 1.0** | 62+-1.2 | 118 |

| | | | |
|---|---|---|---|
| * - Oral intake | | | |
| ** - Administered by injection | | | |

Results of the experiment show that the best results were achieved when higher dosages were administered to the animals. The third test group which orally consumed the peptide at a dose of 10.0 µg/kg of living tissue, have experienced weight gain of 30% higher then the control group. The sixth group which received the peptide in injection form at a dose of 1.0 µg/kg of living tissue, have experienced weight gain of 18% higher then the control group.

The experiments showed that the use of the peptide did not cause any negative effects or deviation from the norm in biochemical indicators of the blood of the test animals. The peptide did have the effect of increasing the muscle mass of the test animals.

### Example 12

The example outlines the influence of the peptide on the weight gain of the rooster chicks.

The experiment was conducted on roosters type "P-46" which were destined as a poultry product. The roosters were at the age of 24 hours. The test roosters were divided into 5 groups of 13 chicks each. The first group served as a control group and the other 4 as the test groups. The roosters in the test groups received the peptide mixed in the feed in the quantity of 8 µg/kg living mass for the second group, 12 µg/kg for the third, 16 µg/kg for the fourth and 20 µg/kg for the fifth group.

The chicks where fed until they reached the age of 10 weeks with combined fodder as recommended for these type of poultry.

All the roosters in all the test and control groups have survived the duration of the experiment. Visual observations showed that the test groups have willingly consumed all feed and where of no different appearance then the control group. Table # 11 outlines the details of the experiment.

**Table 11**

| # of test animals | dose of peptide µg/kg | weight gain g. | net weight gain % |
|---|---|---|---|
| 1 | - | 10.2 | 100 |
| 2 | 8 | 10.4 | 102 |
| 3 | 12 | 12.5 | 122.6 |
| 4 | 16 | 11.8 | 115.7 |
| 5 | 20 | 11.6 | 113.7 |

The average weight gain in test groups 3-5 was higher then the control group by 13-23% and the amount of feed has declined at the same rate. The food consumption in test groups 2-5 has proved to be lower than the control group at the rate of 2.4% for group # 2, 17.1% in group # 3, 14.9% for group # 4, and 14.4% for group # 5.

The organoleptic quality of the meat of the roosters in the test groups has shown that the colour, scent, consistency and quality of taste was not different than the meat in the control group of roosters.

### Example 13

The example outlines the influence of the peptide on the weight gain of young male bulls.

For the purposes of this experiment, 7 groups of young bulls were used age 2 months and 12 calves for each test group. The first group served as a control group and the other six were the test groups. The first three test groups received the peptide in a water solution in oral form on empty stomach at a rate of 1.0 µg/kg for the first test group, 5.0 µg/kg for the second test group, and 10.0 µg/kg for the third test group. The other three test groups received the peptide in an injection form in 0.01 water solution in dosages of 0.1 µg/kg for the fourth group, 0.5 µg/kg for the fifth group and 1.0 µg/kg for the sixth group.

All test groups did not display any visible signs of deviation from norm during clinical and physiological observations.

The results which are outlined in table 12 attest to the fact that the peptide is a positive influence on the rate of growth and weight gain in the bulls, when consumed orally and intervenously.

**Table 12**

| # of test group | peptide dose µg/kg | weight gain g | net weight gained % |
|---|---|---|---|
| 1 | - | 24.6 | 100 |
| 2 | 1.0* | 27.2 | 110 |
| 3 | 5.0* | 26.1 | 106 |
| 4 | 10.0* | 34.6 | 142 |
| 5 | 0.1** | 30.3 | 123 |
| 6 | 0.5** | 29.7 | 121 |
| 7 | 1.0** | 37.6 | 153 |

| | | | |
|---|---|---|---|
| * - oral intake | | | |
| ** - intravenous injection | | | |

The highest positive results were gained with the highest dosages.

Upon the administering of the peptide in oral form in water solution at a dose of 10 µg/kg of living mass, the average weight gain for 24 hour period was recorded to be 583 grams which was higher by 172 grams then the control group during 60 days of measurements. When administered in a water solution in an injected form (dose 1 µg/kg of living mass), the results where even higher. The average 24 hour weight gain was 627 g is higher by 217 g then the control group.

### Example 14

This example outlines the influence of the peptide on weight gain in fish.
a) Ten carp fish were placed in an aquarium which contained a peptide solution for the duration of 2 hours (concentration 0.25 mg/l). The fish were individually marked and weighed prior to placement into the recirculating aquarium system. The carp fish received daily dosage of dry food at a dose of 3% of original mass.
After the 20 day period the fish where taken out and weighed. The results of the weighing process are outlined in table # 13.

**Table # 13**

| | Groups | |
|---|---|---|
| Indicators | Control g. | Test g. |
| number of fish | 10 | 10 |
| average mass at the beginning of the test, g. | 31.6+-3.9 | 33.0+-4.9 |
| average mass at end of test period, g. | 33.0+-4.7 | 40.6+-5.6 |
| comparison to control group % | 100 | 123.0 |

The carps which were treated with the peptide have on average gained 23.0% weight with individual deviations of 18.9 to 31.0%.
b) This experiment was conducted with a young trout having 0.5 g weight. The fish where immersed in a solution with a peptide concentration of 1.0 µg/l during 2 months. The results showed that the average weight of the fish in the control group was g and in the test groups was 3.86 grams.

Therefore the growth of the test groups in relation to the control group was at a rate of 36.4%.

### Example 15

The pharmaceutical composition on basis of a active component as indicated in example # 1 was prepared with a peptide content of 0.001 to 0.1%, preferably with a content of 0.001 to 0.01%.

Water carbohydrates and other carriers were used in compositions which were used in food supplements and liquids for the animals.

The preparation of the food supplements was achieved by mixing the peptide powder with water or food. The amount of food to be added to the mixture depends on the desired consistency or the conditions of the experiment.
a) In the experiments with fishes, the fishes were swam in a water solution with a peptide concentration of 1.0 mg/L.
b) The pharmaceutical composition on the basis of the biologically active component was received as indicated in example # 1. The composition was prepared with a peptide content of 0.001% for mixture which was added to the drinking solution of the calves. The mixing agent was water.
c) The pharmaceutical composition on the basis of the biologically active component was received as indicated in example # 1. The composition was prepared with a peptide content of 0.001% for the mixture which was added to the drinking solution of the piglets. The mixing agent was water.

### Commercial application

The peptides and the pharmaceutical composition in accordance with examples # 5-14 can be widely used in medicine and agriculture. The most useful peptides have the following formula: X-Tyr-Y-Phe-Z-A wherein X is Arg, D-Arg, Orn, D-Orn, Lys, D-Lys, Har, D-Har, Cyt, D-Cyt; Y is D-Ala, D-Val, D-Leu, D-Ile, D-Phe, D-Asn, D-Trp, D-Pro, D-Ser, D-Thr, D-Tyr, D-Hyp, D-Cys, D-CysCys, D-Met, D-Lys, D-Har, D-Arg, D-His, D-Asp, D-Glu, D-β-Ala, D-Orn; Z is D-Ala, D-Val, D-Leu, D-Ile, D-Phe, Asn, D-Asn, Gly, Gln, D-Gln, D-Trp, D-Pro, D-Ser, D-Thr, D-Tyr, Hyp, D-Hyp, Cys, D-Cys, Cys-Cys, Cys-D-Cys, D-Cys-Cys, D-Cys-D-Cys, D-Met, Lys, D-Lys, Arg, D-Arg, His, D-His, Asp, D-Asp, Glu, D-Glu, B-Ala, D-β-Ala, Orn, D-Orn; and A is OH or a substituted amide (C₁-C₃).

Commercial useful pharmaceutical compositions comprise an active ingredient as described above and a diluent or filler.

## Claims

1. A peptide of formula I X-Tyr-Y-Phe-Z-A I wherein
X is arginine, D-arginine, ornithine, D-ornithine, lysine, D-lysine, homoarginine, D-homoarginine, citrulline, or D-citrulline;
Tyr is tyrosine;
Y is D-alanine, D-valine, D-leucine, D-isoleucine, D-phenylalanine, D-asparagine, D-tryptophan, D-proline, D-serine, D-threonine, D-tyrosine, D-hydroxyproline, D-cysteine, D-cysteyl-cysteine, D-methionine, D-lysine, D-homoarginine, D-arginine, D-histidine, D-aspartic acid, D-glutamic acid, D-β-alanine, or D-ornithine;
Phe is phenylalanine;
Z is D-alanine, D-valine, D-leucine, D-isoleucine, D-phenylalanine, asparagine, D-asparagine, glycine, glutamine, D-glutamine, D-tryptophan, D-proline, D-serine, D-threonine, D-tyrosine, hydroxyproline, D-hydroxyproline, cysteine, D-cysteine, cysteyl-cysteine, cysteine-D-cysteine, D-cysteyl-cysteine, D-cysteine-D-cysteine, D-methionine, lysine, D-lysine, arginine, D-arginine, histidine, D-histidine, aspartic acid, D-aspartic acid, glutamic acid, D-glutamic acid, β-alanine, D-β-alanine, ornithine, or D-ornithine; and
A is hydroxyl or substitued amide (C₁-C₃).

2. The peptide of claim 1 wherein A is hydroxyl.

3. The peptide as claimed in claim 1 wherein
X is arginine, D-arginine, D-ornithine, homoarginine, D-homoarginine, or citrulline;
Y is D-ornithine, D-alanine, or D-arginine;
Z is D-alanine, glycine, D-proline or β-alanine; and
A is hydroxyl or substitued amide (C₁-C₃).

4. The peptide of claim 3 wherein A is hydroxyl.

5. The peptide as claimed in claim 1 wherein
X is arginine, homoarginine, D-homoarginine, citrulline, or D-citrulline;
Y is D-alanine, D-ornithine, or D-arginine;
Z is D-alanine; and
A is hydroxyl or substitued amide (C₁-C₃).

6. The peptide of claim 5 wherein A is hydroxyl.

7. The peptide of the formula I as claimed in claim 1, wherein X is arginine; Y is D-alanine; Z is D-proline or β-alanine and A is hydroxyl.

8. A peptide as claimed in claim 1 consisting essentially of the sequence H-D-Orn-Tyr-D-Ala-Phe-D-Ala-OH, H-Arg-Tyr-D-Ala-Phe-β-Ala-OH, H-Arg-Tyr-D-Ala-Phe-Gly-OH, H-Arg-Tyr-D-Ala-Phe-D-Pro-OH, H-Arg-Tyr-D-Ala-Phe-D-Ala-OH, H-Arg-Tyr-D-Orn-Phe-D-Ala-OH, or

H-Arg-Tyr-D-Arg-Phe-D-Ala-OH.

9. A peptide consisting essentially of the sequence: H-D-Arg-Tyr-D-Arg-Phe-D-Ala-NH₂ H-Har-Tyr-D-Arg-Phe-D-Ala-NH₂, H-D-Har-Tyr-D-Arg-Phe-D-Ala-NH2, H-Cyt-Tyr-D-Arg-Phe-D-Ala-NH2, or

H-D-Cyt-Tyr-D-Arg-Phe-D-Ala-NH2.

10. A peptide as claimed in claim 1 consisting essentially of the sequence H-Arg-Tyr-D-Ala-Phe-Gly-OH or H-Arg-Tyr-D-Orn-Phe-D-Ala-OH

11. A peptide as claimed in claim 1 consisting essentially of the sequence H-Arg-Tyr-D-Ala-Phe-Gly-OH.

12. A composition comprising one or more peptides as claimed in any preceding claim 1 to 11 and a pharmaceutically acceptable carrier or diluent.

13. A composition as claimed in claim 12, wherein the composition comprises from 0.001 weight percent to 0.1 weight percent of the peptide.

14. A substance or composition as claimed in any preceding claim 1 to 13 for use as a medicament or food supplement or pharmaceutical composition.

15. Use of a substance or composition as claimed in any preceding claim 1 to 14 for preparing a pharmaceutical composition or medicament or food supplement for modulating one or more physiological processes selected from the group consisting of weight gain, activities of the epithelium growth zone, hair growth, wound healing, and reparative and anabolic processes.

16. Use of a substance or composition as claimed in any preceding claim 1 to 14 for preparing a medicament for relieving pain.

17. Use of a substance or composition as claimed in any preceding claim 1 to 14 for preparing a medicament or food supplement for increasing weight gain in an animal.

## Patentansprüche

1. Peptid der Formel I X-Tyr-Y-Phe-Z-A (I) worin ist:
X Arginin, D-Arginin, Ornithin, D-Ornithin, Lysin, D-Lysin, Homoarginin, D-Homoarginin, Citrullin oder D-Citrullin;
Tyr Tyrosin:
Y D-Alanin, D-Valin, D-Leucin, D-Isoleucin, D-Phenylalanin, D-Asparagin, D-Tryptophan, D-Prolin, D-Serin, D-Threonin, D-Tyrosin, D-Hydroxyprolin, D-Cystein, D-Cysteyl-cystein, D-Methionin, D-Lysin, D-Homoarginin, D-Arginin, D-Histidin, D-Asparaginsäure, D-Glutaminsäure, D-β-Alanin oder D-Ornithin;
Phe Phenylalanin;
Z D-Alanin, D-Valin, D-Leucin, D-Isoleucin, D-Phenylalanin, Aspargin, D-Aspargin, Glycin, Glutamin, D-Glutamin, D-Tryptophan, D-Prolin, D-Serin, D-Threonin, D-Tyrosin, Hydroxyprolin, D-Hydroxyprolin, Cystein, D-Cystein, Cysteyl-cystein, Cystein-D-cystein, D-Cysteyl-cystein, D-Cystein-D-cystein, D-Methionin, Lysin, D-Lysin, Arginin, D-Arginin, Histidin, D-Histidin, Asparaginsäure, D-Asparaginsäure, Glutaminsäure, D-Glutaminsäure, β-Alanin, D-β-Alanin, Ornithin oder D-Ornithin; und
A Hydroxyl oder substituiertes Amid (C₁-C₃).

2. Peptid nach Anspruch 1, worin A Hydroxyl ist.

3. Peptid nach Anspruch 1, worin ist:
X Arginin, D-Arginin, D-Ornithin, Homoarginin, D-Homoarginin oder Citrullin;
Y D-Ornithin, D-Alanin oder D-Arginin;
Z D-Alanin, Glycin, D-Prolin oder β-Alanin; und
A Hydroxyl oder substituiertes Amid (C₁-C₃).

4. Peptid nach Anspruch 3, worin A Hydroxyl ist.

5. Peptid nach Anspruch 1, worin ist:
X Arginin, Homoarginin, D-Homoarginin, Citrullin oder D-Citrullin;
Y D-Alanin, D-Ornithin, oder D-Arginin;
Z D-Alanin; und
A Hydroxyl oder substituiertes Amid (C₁-C₃).

6. Peptid nach Anspruch 5, worin A Hydroxyl ist.

7. Peptid der Formel I nach Anspruch 1, worin X Arginin ist, Y D-Alanin, Z D-Prolin oder β-Alanin und A Hydroxyl.

8. Peptid nach Anspruch 1, bestehend im Wesentlichen aus der Sequenz H-D-Orn-Tyr-D-Ala-Phe-D-Ala-OH, H-Arg-Tyr-D-Ala-Phe-β-Ala-OH, H-Arg-Tyr-D-Ala-Phe-Gly-OH, H-Arg-Tyr-D-Ala-Phe-D-Pro-OH, H-Arg-Tyr-D-Ala-Phe-D-Ala-OH, H-Arg-Tyr-D-Orn-Phe-D-Ala-OH oder

H-Arg-Tyr-D-Arg-Phe-D-Ala-OH.

9. Peptid, bestehend im Wesentlichen aus der Sequenz in Anspruch 1 H-Arg-Tyr-D-Arg-Phe-D-Ala-NH2 H-Har-Tyr-D-Arg-Phe-D-Ala-NH2 H-D-Har-Tyr-D-Arg-Phe-D-Ala-NH2 H-Cyt-Tyr-D-Arg-Phe-D-Ala-NH2 oder

H-D-Cyt-Tyr-D-Arg-Phe-D-Ala-NH2.

10. Peptid nach Anspruch 1, bestehend im Wesentlichen aus der Sequenz H-Arg-Tyr-D-Ala-Phe-Gly-OH oder H-Arg-Tyr-D-Orn-Phe-D-Ala-OH.

11. Peptid nach Anspruch 1, bestehend im Wesentlichen aus der Sequenz H-Arg-Tyr-D-Ala-Phe-Gly-OH.

12. Zusammensetzung, umfassend ein oder mehrere Peptide nach irgendeinem der vorhergehenden Ansprüche 1 bis 11 und ein pharmazeutisch akzeptabler Träger oder Verdünner.

13. Zusammensetzung nach Anspruch 12, wobei die Zusammensetzung 0,001 Gew.% bis 0,1 Gew.% Peptid enthält.

14. Subtanz oder Zusammensetzung nach irgendeinem der Ansprüche 1 bis 13 zur Verwendung in einem Medikament oder in einem Nahrungsmittelzusatz oder in einer pharmazeutischen Zusammensetzung.

15. Verwendung einer Subtanz oder einer Zusammensetzung nach irgendeinem der vorhergehenden Ansprüche 1 bis 14 zur Herstellung einer pharmazeutischen Zusammensetzung oder eines Medikaments oder eines Nahrungsmittelzusätzes zur Modulierung ein oder mehrerer physiologischer Vorgänge ausgewählt aus der Gruppe Gewichtszunahme, Aktivitäten der Epithel-Wachstumszone, Haarwachstum, Wundheilung, reparative und anabole Prozesse.

16. Verwendung einer Substanz oder einer Zusammensetzung nach irgendeinem der Ansprüche 1 bis 14 zur Herstellung eines Medikaments zur Schmerzlinderung.

17. Verwendung einer Substanz oder einer Zusammensetzung nach irgendeinem der Ansprüche 1 bis 14 zur Herstellung eines Medikaments oder eines Nahrungsmittelzusatzes zur Erhöhung der Gewichtszunahme in einem Tier.

## Revendications

1. Peptide de formule I : X-Tyr-Y-Phe-Z-A I dans lequel
X est l'arginine, la D-arginine, l'ornithine, la D-ornithine, la lysine, la D-lysine, l'homoarginine, la D-homoarginine, la citrulline, ou la D-citrulline ;
Tyr est la tyrosine ;
Y est la D-alanine, la D-valine, la D-leucine, la D-isoleucine, la D-phénylalanine, la D-asparagine, le D-tryptophane, la D-proline, la D-sérine, la D-thréonine, la D-tyrosine, la D-hydroxyproline, la D-cystéine, la D-cystéyl-cystéine, la D-méthionine, la D-lysine, la D-homoarginine, la D-arginine, la D-histidine, l'acide D-aspartique, l'acide D-glutamique, la D-β-alanine, ou la D-ornithine ;
Phe est la phénylalanine;
Z est la D-alanine, la D-valine, la D-leucine, la D-isoleucine, la D-phénylalanine, l'asparagine, la D-asparagine, la glycine, la glutamine, la D-glutamine, le D-Tryptophane, la D-proline, la D-sérine, la D-thréonine, la D-tyrosine, l'hydroxyproline, la D-hydroxyproline, la cystéine, la D-cystéine, la cystéyl-cystéine, la cystéine-D-cystéine, la D-cystéyl-cystéine, la D-cystéine-D-cystéine, la D-méthionine, la lysine, la D-lysine, l'arginine, la D-arginine, l'histidine, la D-histidine, l'acide aspartique, l'acide D-aspartique, l'acide glutamique, l'acide D-glutamique, la β-alanine, la D-β-alanine, l'ornithine, ou la D-ornithine ; et
A est un groupe hydroxyle ou un amide substitué (C₁ à C₃).

2. Peptide selon la revendication 1, dans lequel A est un groupe hydroxyle.

3. Peptide selon la revendication 1, dans lequel
X est l'arginine, la D-arginine, la D-ornithine, l'homoarginine, la D-homoarginine, ou la citrulline ;
Y est la D-ornithine, la D-alanine, ou la D-arginine ;
Z est la D-alanine, la glycine, la D-proline ou la β-alanine ; et
A est un groupe hydroxyle ou un amide substitué (C₁ à C₃).

4. Peptide selon la revendication 3, dans lequel A est un groupe hydroxyle.

5. Peptide selon la revendication 1, dans lequel
X est l'arginine, l'homoarginine, la D-homoarginine, la citrulline ou la D-citrulline ;
Y est la D-alanine, la D-ornithine ou la D-arginine ;
Z est la D-alanine ; et
A est un groupe hydroxyle ou un amide substitué (C₁ à C₃).

6. Peptide selon la revendication 5, dans lequel A est un groupe hydroxyle.

7. Peptide de formule I selon la revendication 1, dans lequel X est l'arginine ; Y est la D-alanine ; Z est la D-proline ou la β-alanine et A est un groupe hydroxyle.

8. Peptide selon la revendication 1, essentiellement constitué de la séquence : H-D-Orn-Tyr-D-Ala-Phe-D-Ala-OH, H-Arg-Tyr-D-Ala-Phe-β-Ala-OH, H-Arg-Tyr-D-Ala-Phe-Gly-OH, H-Arg-Tyr-D-Ala-Phe-D-Pro-OH, H-Arg-Tyr-D-Ala-Phe-D-Ala-OH, H-Arg-Tyr-D-Orn-Phe-D-Ala-OH, ou

H-Arg-Tyr-D-Arg-Phe-D-Ala-OH,

9. Peptide essentiellement constitué de la séquence : H-D-Arg-Tyr-D-Arg-Phe-D-Ala-NH2, H-Har-Tyr-D-Arg-Phe-D-Ala-NH₂, H-D-Har-Tyr-D-Arg-Phe-D-Ala-NH2, H-Cyt-Tyr-D-Arg-Phe-D-Ala-NH₂, ou

H-D-Cyt-Tyr-D-Arg-Phe-D-Ala-NH₂,

10. Peptide selon la revendication 1, essentiellement constitué de la séquence H-Arg-Tyr-D-Ala-Phe-Gly-OH ou H-Arg-Tyr-D-Orn-Phe-D-Ala-OH.

11. Peptide selon la revendication 1, essentiellement constitué de la séquence H-Arg-Tyr-D-Ala-Phe-Gly-OH.

12. Composition comprenant un ou plusieurs peptides selon l'une quelconque des revendications précédentes 1 à 11, et un vecteur ou un diluant pharmaceutiquement acceptable.

13. Composition selon la revendication 12, dans laquelle la composition comprend de 0,001 % en poids à 0,1 % en poids du peptide.

14. Substance ou composition selon l'une quelconque des revendications précédentes 1 à 13, destinée à être utilisée comme médicament ou supplément alimentaire ou composition pharmaceutique.

15. Utilisation d'une substance ou d'une composition selon l'une quelconque des revendications précédentes 1 à 14, pour la préparation d'une composition pharmaceutique ou d'un médicament ou d'un supplément alimentaire destiné(e) à moduler un ou plusieurs processus physiologiques choisis dans le groupe constitué par la prise de poids, les activités de la zone de croissance de l'épithélium, la croissance pilleuse, la cicatrisation des plaies, et les processus réparateurs et anaboliques.

16. Utilisation d'une substance ou d'une composition selon l'une quelconque des revendications précédentes 1 à 14, pour la préparation d'un médicament destiné à soulager la douleur.

17. Utilisation d'une substance ou d'une composition selon l'une quelconque des revendications précédentes 1 à 14, pour la préparation d'un médicament ou d'un supplément alimentaire destiné à faire augmenter la prise de poids chez un animal.
